(19) Europäisches Patentamt

European Patent Office

Office européen des brevets

(11) **EP 1 432 349 B1**

(12) **EUROPEAN PATENT SPECIFICATION**

(45) Date of publication and mention
of the grant of the patent:
**21.06.2006 Bulletin 2006/25**

(51) Int Cl.:
*A61B 5/0444* (2006.01)

(21) Application number: **02765054.8**

(22) Date of filing: **27.09.2002**

(86) International application number:
**PCT/GB2002/004410**

(87) International publication number:
**WO 2003/028550 (10.04.2003 Gazette 2003/15)**

(54) **APPARATUS FOR MONITORING FETAL HEART-BEAT**

GERÄT ZUR BEOBACHTUNG DES FÖTALEN HERZSCHLAGS

APPAREIL DE CONTROLE DU RYTHME CARDIAQUE FOETAL

(84) Designated Contracting States:
**AT BE BG CH CY CZ DE DK EE ES FI FR GB GR IE IT LI LU MC NL PT SE SK TR**

(30) Priority: **03.10.2001 GB 0123772**

(43) Date of publication of application:
**30.06.2004 Bulletin 2004/27**

(60) Divisional application:
**06007088.5**

(73) Proprietor: **Qinetiq Limited
Farnborough
Hampshire GU14 OLX (GB)**

(72) Inventors:
  • **SMITH, Mark, John,
QinetiQ Limited
Malvern,
Worcestershire WR6 5EG (GB)**
  • **PENNEY, Richard, W.,
QinetiQ Limited
Malvern,
Worcestershire WR6 5EG (GB)**

(74) Representative: **Bowdery, Anthony Oliver
QinetiQ Limited
Intellectual Property,
Cody Technology Park,
Ively Road
Farnborough, Hants GU14 0LX (GB)**

(56) References cited:
  **US-A- 6 032 072          US-A- 6 067 467**

  • SPENCER, J.A.D.: "Current methods of continuous fetal heart rate monitoring" PROFESSIONAL NURSE, vol. 8, no. 3, December 1992 (1992-12), pages 173-175, XP001121252
  • IKEDA S, TOYAMA K: "Independent component analysis for noisy data - MEG data analysis" NEURAL NETWORKS, vol. 13, 2000, pages 1063-1074, XP002234357
  • CALLAERTS D, SANSEN W, VANDEWALLE J, VANTRAPPEN G, JANSSENS J: "Description of real-time system to extract the fetal electrocardiogram" CLIN. PHYS. PHYSIOL. MEAS., vol. 10, Suppl. B, 1989, pages 7-10, XP009007418
  • IEEE TRANSACTIONS ON BIOMEDICAL ENGINEERING, vol. 47, no. 5, May 2000 (2000-05), pages 567-572, XP002234358 cited in the application
  • LIANG H: "Adaptive independent component analysis of multichannel electrogastrograms" MEDICAL ENGINEERING & PHYSICS, vol. 23, no. 2, March 2001 (2001-03), pages 91-97, XP002234359

EP 1 432 349 B1

**Description**

This invention relates to the field of medical electrocardiographs, in particular to those adapted to monitor non-invasively the heart-beat of an unborn fetus.

The electrocardiogram (ECG) is a key tool in the diagnosis of heart disease and abnormalities in both children and adults. The heart-beat is instigated and controlled by electrical conduction through the heart. In a healthy human a certain characteristic sequence of electrical impulses, which is repeatedly cycled, controls the heart-beat. If voltage sensors are placed on a patient's chest then the electrical activity, and its variation from beat to beat, can be detected and displayed. This is the basis behind the ECG. Alternative detection tools such as Magnetic Resonance Imaging (MRI) and Ultrasound may complement the ECG in providing an understanding of the physiology of the heart, but it is the ECG that importantly indicates the detail of the cardiac rhythm.

The unique detection capability provided by the ECG gives it a very important role to play in the diagnosis and management of abnormal cardiac rhythms, and accordingly it is widely used in hospitals throughout the world. An ECG can, for example, help with the diagnosis of the causes of chest pain and breathlessness and is crucial to the proper use of thrombolysis in treating myocardial infarction. ECG equipment is moreover generally cheaper, more portable and simpler to use than apparatus used for alternative monitoring techniques such as MRI and ultrasound. It therefore does not require such highly trained personnel to operate it and ECG readings can be taken over extended periods (e.g. 24 hours) even whilst the subject is ambulatory.

It is anticipated that the ability to acquire routinely a fetal ECG (fECG) would have at least a similar clinical value to the unborn child as the ECG currently has to children and adults. In addition it is expected to contribute to the early detection and monitoring of ischaemia and heart arrhythmias and abnormalities that might lead to premature death or long-term damage. Unfortunately, as in any non-invasive fetal observation technique, the situation is complicated by the need to extract weaker fetal information from a composite signal containing data relating to both the fetus and the mother. It is significant that subtle cardiovascular indications of dysfunction may be present well before the 20th week of pregnancy, but these cannot be detected until further into the gestation period by currently-used techniques.

The problem in multiple pregnancies is further compounded not only by the need to acquire at least two fetal indications from the composite signal, but also by the fact that accurate detection of cardiac development is of far greater importance to this high risk group. Multiple pregnancies face substantially increased risks of perinatal mortality and morbidity compared with singletons and it is therefore envisaged that antenatal acquisition of their fECGs will have correspondingly greater clinical application. For example monozygous twins face a risk of 3.6% of congenital heart disease. Twin-Twin Transfusion Syndrome (TTTS) is a complication unique to monochorionic twins in which one fetus (the recipient) receives too much blood through the shared placenta at the expense of the other (the donor). Cardiac overload is a typical feature exhibited by the recipient fetus, which would be readily detectable by ECG. TTTS complicates 15% of monochorionic twins and accounts for 17% of all perinatal mortality in twins. In addition, growth restriction, which complicates 5% of singleton pregnancies, affects 25% of dichorionic and 42% of monochorionic twins. In comparison with traditional methods of fetal monitoring, it is envisaged that fECG will offer earlier detection of ischaemia or cardiac compromise, and will provide useful information on the physiological responses to divergent blood volume loads found in TTTS.

Current fetal monitoring tools such as Doppler ultrasound and cardiotocography (CTG) lack sensitivity and specificity. Diagnostic use of CTG appears to have no significant effect on perinatal mortality or morbidity in high-risk pregnancies. Indeed in the Cochrane database there is a trend towards increased perinatal mortality (odds ratio 2.85, 95% confidence interval 0.99 to 7.12) in those assessed by CTG. Although the use of Doppler ultrasound in high-risk pregnancies appears to improve a number of obstetric care outcomes and appears promising in helping to reduce perinatal deaths, it has not been shown to be of benefit in low-risk populations.

The limitations of these techniques, combined with the potential benefits that are offered by fECG has prompted the more recent development of both invasive and non-invasive techniques to record fECGs.

invasive techniques involve the direct attachment of an electrode to the scalp of the baby during labour. The use of scalp electrodes has however increased risks of perinatal infection and so, despite a demonstrable reduction in birth asphyxia, is limited in many countries, including the UK. Such techniques do serve however to indicate the importance of acquiring a detailed fECG recording. In The Lancet, Vol 358, pp 534-8 (2001), Amer-Wahlin *et al* showed that monitoring the ST segment of a fetal heart pulse during labour provided a useful diagnostic indicator of hypoxia. There is therefore a demonstrable need for the ability to obtain reliably this level of fECG detail non-invasively.

A variety of non-invasive techniques have been tested. It was shown as long ago as 1906 that the electrical activity of the heart of the unborn fetus can be detected non-invasively at the surface of the maternal abdomen. Electrodes can therefore be used to detect a composite signal containing information relating to the cardiac activity of both the mother and fetus.

However the amplitude of the maternal signal is typically around 100$\mu$V or more at the abdomen, whereas that of the fetus is of only 10-20 $\mu$V, and may even be less depending on the position of the electrodes on the abdomen and on the presentation and gestation of the fetus. Clearly, to provide useful information, it is necessary to separate the detailed fECG from the composite signal in which it is significantly masked by both the maternal signal and noise.

Many attempts have been made to obtain a meaningful fECG signal from the composite signal detected at the maternal abdomen. D. Callaerts in his PhD thesis "Signal separation methods based on Singular Value Decomposition and their application to real-time extraction of the fetal electrocardiogram from cutaneous recordings", Katholieke Universiteit Leuven, December 1989 describes one method of processing data collected using dedicated hardware equipment. This technique however requires electrodes to be placed along three mutually orthogonal axes intersecting at the location of the fetal heart, in addition to those required to be placed on the mother's chest. This not only means that electrodes must be placed on the mother's back as well as her abdomen, adding to her discomfort, but it also requires an *a priori* knowledge of the position of the fetus e.g. acquired by use of ultrasound.

De Lathauwer *et al.* in "Fetal electrocardiogram by Blind Source Subspace Separation" IEEE Trans. Biomed. Eng. 47(5) 567-572 (2000), apply an improved algorithm to data previously recorded by Callaerts and coworkers. Callaerts reported analysis of the composite data using an algorithm known as Singular Value Decomposition (SVD). De Lathauwer demonstrated that Blind Signal. Separation (BSS) algorithms based on Independent Component Analysis (ICA) can be more effective in separating out the fetal signal in those cases for which the earlier SVD technique was found to work. Potential insights on or improvements to the recording equipment or its configuration were not addressed.

Spencer *et al.* in "Antenatal abdominal fetal electrocardiogram recording - preliminary results of a compact and portable monitor" (Abstract) XVI FIGO World Congress of Gynaecology and Obstetrics, Washington (2000) report results for fetal separation again using purpose-built equipment. However the quality of the separated signals is very poor, with the interval between successive beats in the fECG being discernable in only 59% of cases. Given the level of noise, it is highly unlikely that this equipment could be adapted for effective monitoring of detailed fECGs in all but singleton ; pregnancies. Indeed the emphasis of such devices is not upon acquisition of detail at all, but only on the determination of the fetal heart rate. This is a far simpler measurement to extract. Based on what is known about ECGs taken in children and adults it is expected that more detailed examination of the cardiac rhythm will yield further important diagnostic parameters.

In summary therefore currently-available non-invasive techniques for obtaining the fetal electrocardiogram are generally only of limited effect, even in singleton pregnancies, and / or require a complicated arrangement of electrodes for the recording, making it impossible to be taken by all but highly trained staff. There is a perceived need to provide apparatus for the recording of fetal ECGs from electrodes on the maternal abdomen which is capable of reliably extracting the detailed fetal signal and which admits of more straightforward application than the aforementioned prior art apparatus. It is an object of this invention to provide such apparatus. In particular, it is an object of this invention to provide fECG equipment which is capable of extracting fECGs during multiple pregnancies; this being one of the high-risk groups most likely to gain from the kind of information that a detailed fECG might have to offer.

Accordingly the present invention provides apparatus for the detection of a fetal electrocardiogram (fECG), the apparatus comprising a:plurality of electrodes for application to a patient and signal processing means for deriving an fECG from signals developed by the electrodes, characterised in that:

a) the electrodes are low-noise electrodes for abdominal skin placement during pregnancy and have associated leads with screening to reduce noise level and being in number sufficient to enable at least six signal sources to be monitored; and

b) the signal processing means is a low-noise signal processing means, is connectable to the electrodes, has sufficient sensitivity to detect signals of magnitude comparable with electroencephalography signals, and is arranged to:

i) detect signals developed by the electrodes; and

ii) process electrode signals to obtain data and use a Blind Source Separation technique to process the data and distinguish independent source contributions thereto and derive at least one source signal relating to a single fetus fECG.

This invention is capable of extracting improved fECGs as compared to the prior art. It makes use of improved hardware for significant reduction of unwanted noise in the raw input data and appropriate digital signal processing for separation of the desired fetal signal. Once the electrical noise is reduced in this way (in one or more aspects), the contributions due to maternal muscle noise are seen to be surprisingly high. Once this has been noticed however, the invention provides the advantage of being able to observe objectively the relaxation state of the mother. Previously, in practical application of fetal monitoring equipment, it had been almost impossible to judge, even by the mother, a degree of relaxation below a certain level. Nor was it possible, with all other contributing noise factors, to realise the importance of providing such relaxation. Now, the noise on each signal channel can be observed prior to recording and recording is only begun when the mother is sufficiently relaxed. Accordingly, use of this invention will be of enormous benefit to operators of fECG equipment in judging when best to make a recording. Once the quality of recording is improved, more information can be extracted from the fECG, including respective fECGs in multiple pregnancies. In addition the likelihood of making a successful recording when the fECG signal is weak is greatly increased. Not only is the signal weak at early gestation, as might be expected, but also in the 27 to about 32 week gestation period. This phenomenon is thought to be due to a non-conducting layer forming around the fetus during this period. In any event the effect on fetal ECGs is well-known and discussed in detail by Oostendorp in his PhD thesis "Modelling the fetal ECG", University of Nijmegen, January 1989. This equipment is consequently better able to extract fECGs at early gestation and in the problem area at around 27 to 32 weeks gestation than prior art fECG monitors.

The electrodes are preferably sufficient in number to enable at least eight signal sources to be monitored.

The signal processing means may be arranged:

a) to record signals indicative of voltages developed between pairs of electrodes in a plurality of signal channels;

b) to process digitised data derived from electrode voltages in order to generate at least one source signal which relates to the fECG of a single fetus; and

c) on identification of the at least one source signal relating to the single-fetus fECG, to reconstruct, for at least one signal channel, that component of the digitised data within the channel which is attributable to that fetus, and which therefore corresponds to a single-channel fECG.

The signal processing means may be arranged to process the data in digitised form to generate a plurality of separated source signals, at least one of which relates to the fECG of a single fetus.

Preferably the electrodes may be placed on the skin such that skin impedance at each electrode is less than $5k\Omega$ and, ideally, $2k\Omega$.

It is preferred that the signal processing means comprises electronic components for processing analogue voltage signals developed between pairs of electrodes to provide digital signals in the plurality of signal channels and data processing means to process the digital signals.

The electronic components most preferably comprise, for each abdominal electrode, a low-noise differential amplifier for amplifying the difference between each electrode's voltage signal and a signal derived from the voltage developed at the reference electrode, an anti-aliasing low-pass filter and an analogue to digital converter. The analogue to digital converter is preferably a simultaneous multi-channel A/D converter which enables simultaneous sampling (and therefore synchronous digitisation) of the signals from each electrode.

These electronic components may be located in a multichannel lead box, remote from the patient, and connectable to the electrodes by, for each electrode, respective screened leads. This multichannel lead box may be one that is suitable for use in taking electroencephalography (EEG) scans. Additionally, a pre-amplifier may be located adjacent each electrode. This amplifies each signal to some extent before it is transmitted along the leads, decreasing relative losses and making it more robust to the effects of noise.

Alternatively, the lead box may be located near to a patient and in communication with the data processing means over a wireless link. This gives the mother considerably more freedom of movement while connected for a scan.

Consideration of the noise level is important, but its criticality depends on the stage of gestation and also whether or not there is more than one fetus present. All the low-noise factors listed above contribute to the possibility of providing input signal channels in practice with a noise component of less than $10\mu V$ and, ideally, less than $3\mu V$. Prior art devices, without such noise-reducing features, have only ever been able to obtain fECGs for singletons, after about 20 weeks of gestation. By way of contrast, prototype apparatus built in accordance with this invention has demonstrated extraction of fECGs for triplets at 20 weeks gestation, twins at 18 weeks and singletons at 15 weeks.

The low-noise electrodes preferably comprise a plurality of electrodes for placement on the skin in the abdominal area and a common reference electrode, and the signal processing means is arranged to record signals indicative of voltages developed between each abdominal electrode and the reference electrode. They preferably further include a low-noise electrode which is connected to earth.

The taking of voltage readings with reference to a common electrode is referred to as a unipolar configuration. Prior art techniques have always used a bipolar configuration. That is, measurements are taken between multiple pairs of electrodes. The electrode pairs in the bipolar configuration are placed sufficiently close that it may be assumed that the same noise contribution is made to each channel. By taking a difference signal therefore, the noise is reduced. Of course this relies on the signal being different in closely-spaced electrodes. A unipolar configuration is inherently more sensitive to the signal itself but consideration of the noise of the system has actively discouraged any attempt to make use of unipolar readings. One skilled in the art has hitherto believed that, without the noise reduction offered by a bipolar configuration, it would be impossible to extract a fetal ECG from a composite signal. It is only with development of apparatus in accordance with the present invention that this long-held belief has been proved erroneous.

A unipolar configuration offers advantages to both data processing and, rather unexpectedly, to noise reduction. Most prior art processing techniques to date have been based on the use of SVD or Principal Component Analysis to achieve source separation. Both these techniques require bipolar readings to be input, which, in turn, places constraints on the geometry of the electrodes used to gather the data. Data collected using a unipolar configuration is however open to analysis by further separation algorithms, for example, ICA. Thus there is an increased flexibility afforded the signal processing by the present invention, with the added advantage that equipment may be operated successfully by a less expert user.

Unipolar signal channels allow an observer to view directly both the signal and noise on the channel. It is therefore far easier to recognise the noise component and to observe its level. If it can be made to fall, the reduction is readily observed. Thus, in a practical application of the apparatus according to this aspect of the invention, given the fact that maternal muscle noise makes a significant contribution to overall system noise, it is easier to see if the mother is relaxed prior to taking any measurements. In this way, recording quality can be considerably improved and significant information extracted from fetal ECGs, even in multiple pregnancies and at early gestation.

Moreover, unipolar channels, as provided in the present apparatus, are digitised and then available for processing electronically by whatever means is required. This allows software to be used to replicate a bipolar system by calculating differences between respective pairs of unipolar electrode channels. This also enables the same hardware to be used, for example, to measure a conventional ECG..

If more than one source signal relates to the same fetal fECG, the signal processing means may be arranged, on identification of the more than one source signal relating to the single fetus to combine these source signals with appropriate weighting in the reconstruction of the single-channel fECG. In this way the natural variation of the fECG waveform morphology over the maternal abdomen may be observed in a similar way to that in which the ordinary ECG waveform morphology is seen to vary between different chest leads.

In detecting fetal electrocardiograms in a multiple pregnancy, the processing means is preferably arranged, on identification of the at least one source signal relating to each fetus, to reconstruct, for at least one abdominal electrode, components of the digitised data within the corresponding signal channel which is attributable to each fetus. Components of digitised data may be reconstructed for all signal channels, thereby enabling construction of an abdominal surface intensity map of signal strength from each fetus. This may be used to provide an indication of fetal position and so ensure that a given diagnosis is assigned to the correct fetus.

The processing means is preferably arranged to generate the separated source signals by ICA. The capabilities of the ICA algorithm are more readily exploited when unipolar, rather than bipolar, readings are taken. If only unipolar readings are processed, there is no need for any special attention to be paid to electrode arrangement or to fetal presentation. The system is therefore more versatile and routine usage more practical. The standard operator of the fECG apparatus in accordance with this embodiment of the invention may be a person less highly trained than a doctor such as a midwife. Use of unipolar readings is facilitated by the care taken in the reduction of noise and, in particular, muscle noise.

The leads are preferably electrically screened cables, which ideally are kept close together to reduce noise from varying magnetic fields. The electrodes may be self-adhesive and preferably able to resolve signals over a bandwidth which includes 0.5 to 200 Hz. Preferably, the signal channels are arranged to contain a visible noise component on a display apparatus of less than $10\mu V$ and, ideally, less than $3\mu V$. Subsequent digital signal processing and signal separation is arranged thereafter to reduce the noise level in separated fECG signals to values considerably lower than this and certainly sufficient to identify detail in the fetal P and T waves, which are generally around $1\ \mu V$ in amplitude.

In a further aspect the present invention provides a computer-readable medium for use in a method of extraction of one or more fetal electrocardiograms from composite signals derived by signal processing means from signals developed by electrodes applied to a patient, characterised in that in operation the electrodes are applied externally to a pregnant woman's abdominal skin and the computer-readable medium embodies computer-executable instructions comprising program code for controlling computer apparatus to:

a) filter multi-channel digitised composite signals, each signal corresponding to a difference between a voltage developed at a signal electrode and that developed at a reference electrode, in order to remove unwanted frequency components;

b) generate a plurality of separated source signals from the corresponding plurality of filtered composite signals each treated as a linear mixture of unknown source signals;

c) identify, either automatically or by prompting for a user input, a source signal or source signals corresponding to a single-fetus ECG;

d) reconstruct, for each single-fetus ECG identified, and for at least one signal channel, that component of the filtered composite signal within the channel which is attributable to that fetus and therefore corresponds to a single-channel fECG; and

e) display at least one reconstructed fECG to a user.

Embodiments of the invention will now be described by way of example only and with reference to the accompanying drawings, in which:

Figure 1 is an example of a single-channel ECG reading taken from an adult;

Figure 2 is a schematic expanded portion of one cycle of the ECG illustrated in Figure 1 showing conventional labelling of specific features of the heart-beat waveform;

Figure 3 is a schematic illustration of apparatus suitable for recording fECGs in accordance with the present invention;

Figure 4 is a schematic illustration of an arrangement of electrodes on the maternal abdomen suitable for recording fECG signals in accordance with this invention;

Figure 5a is a schematic illustration of apparatus suitable for recording fECGs in accordance with a second embodiment of the present invention;

Figure 5b is a schematic illustration of apparatus suitable for recording fECGs in accordance with a third embodiment of the present invention;

Figure 6 is an exemplary illustration of the composite signal recorded during a singleton pregnancy on the 12 channels of the electrodes of Figure 4;

Figure 7 illustrates an example of data obtained in performing signal analysis on the 12 channels of composite signal of Figure 5;

Figure 8 shows an example of a parameterised average fECG taken using the apparatus of this invention; and

Figure 9 is an illustration of a display generated by a prototype apparatus of the invention for interpretation by an operator.

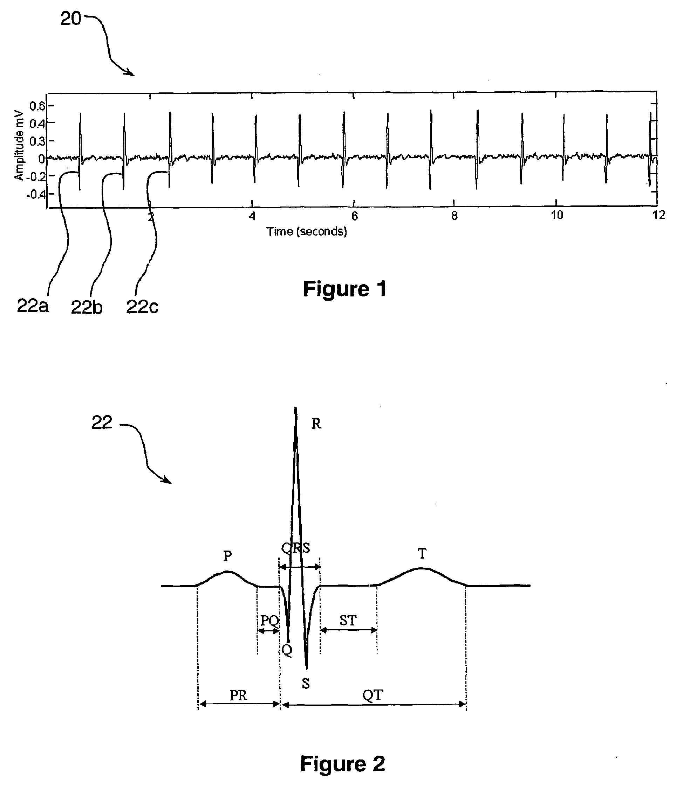

With reference to Figure 1, a typical single-channel adult ECG trace 20 can be seen to comprise a series of regular pulses (22a, 22b, 22c) of amplitude ~ 0.8 mV, one such pulse being produced roughly every 0.75s; that is, around 80 per minute. Each pulse corresponds to a single heart-beat

Figure 2 shows a schematic view of an averaged pulse 22 such as that seen in the trace 20 shown in Figure 1. This is referred to as the underlying cardiac complex which repeats with each beat of the heart. The general waveform of the complex 22 has various features which are known to provide important diagnostic information. These include (in order of appearance during the beat) P, Q, R, S and T waves. The QRS complex corresponds to the principal, powerful beat of the heart. Using this notation the position of the onset of the P and Q waves, and of the termination of the S and T waves in particular can be determined and labelled as indicated in the figure. In this way relative timings and durations of different parts of the cardiac complex are routinely obtained. These can be compared with known equivalents in a healthy heart and diagnosis aided. Parameters with particularly important known diagnostic properties in ordinary ECGs are, for example, PR and QT intervals (periods between onset of P wave and onset of Q wave and between onset of Q and termination of T wave respectively), QRS duration and relative heights of the PQ and ST segments (approximately flat readings between wave features) compared with the isoelectric line.

Extraction of a detailed fECG would provide the ability to display the instantaneous peak-to-peak heart rate in a rhythm strip showing the P and T waves and, in addition, the ability to examine and characterise the detail in the underlying waveform by measuring quantities such as the PR and QT intervals and QRS duration, etc. From comparison with the benefits of ordinary ECGs, it is expected that the former ability would enable the diagnosis of cardiac arrhythmias and anomalies such as atrial or ventricular ectopic beats or heart block. The latter would enable diagnosis of more subtle conditions not manifested directly in the heart-rate such as long-QT syndrome.

It is to be noted that extraction of the detailed fetal ECG from a maternal abdominal signal is clearly a more demanding problem than finding merely the fetal heart-rate after suppressing the maternal QRS peaks. It is necessary to detect the fetal P and T waves in order that a detailed characterisation may be performed.

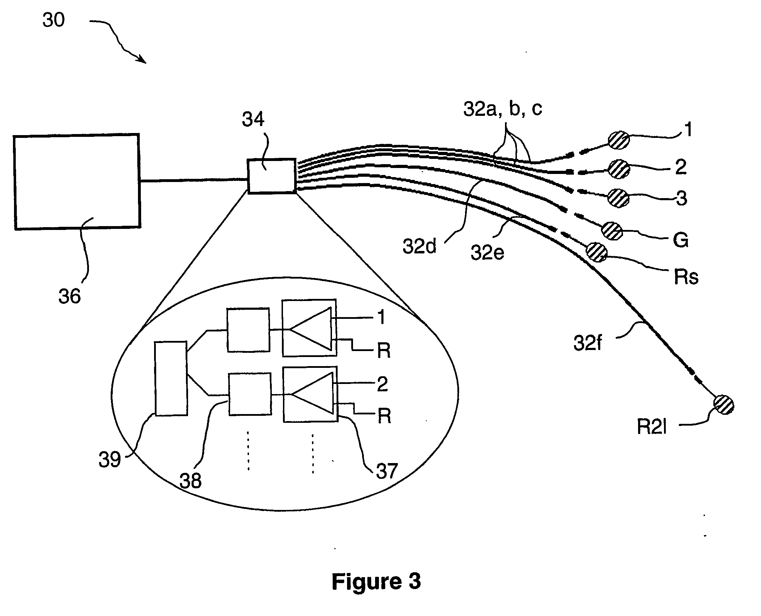

Figure 3 is an illustration of apparatus suitable for implementing this invention, indicated generally by 30. The apparatus 30 comprises a number of electrodes (G, Rs, RI, 1, 2, 3, ...) suitable for attaching to the mother's skin and monitoring voltage signals generated thereon. The electrodes G, Rs, RI, 1, 2, 3, .... are connected via respective screened leads (32a, b, c, d.....) to a lead box 34. At the lead box 34 the signals are amplified and converted to digital readings ready for recording and processing by a computer 36. For convenience only six electrodes G, Rs, RI, 1, 2, 3 are illustrated in this Figure, but in this specific embodiment of the invention there are, in fact, 15 as will become apparent later. Processing electronics within the lead box 34 for the signal electrodes (1, 2, 3,....) are shown inset in Figure 3. These comprise, for each abdominal electrode (1, 2, 3, ....), a low-noise differential amplifier 37 and an anti-aliasing low-pass filter 38 as well as a common (to all electrode channels) simultaneous multi-channel analogue to digital (A/D) converter 39.

Somewhat surprisingly it has been found that a commercially available Electroencephalography (EEG) system has proved suitable for adaptation for acquisition and display of raw input composite fECG data readings. Accordingly the computer 36 is that from a portable EEG system (SYS98-Port24-CL) supplied by Micromed Electronics UK Ltd (Woking, Surrey) and which therefore comprises a battery-powered laptop computer running System '98 EEG recording and analysis software (SYS-98) under Microsoft Windows NT operating system. The SYS-98 software provides a convenient interface from the A/D outputs to display apparatus (screen, not shown) and to a data storage medium (hard disk). Bespoke software is also run on this computer 36, this software being specifically designed to enable reading of recorded (by EEG-specific software) data, separation and processing of the fetal contributions) and for display of the fECG and parameters derived from it (such as fetal heart-rate, PR, QRS, QT intervals, etc.). Details of the processing carried out by this bespoke software will be explained later. The type of computer 36 is clearly not critical however, all that is required is that it has sufficient processing capacity for running the recording, processing and display software and sufficient memory for storing the recorded data, processed results and the display itself. Preferably the computer should be portable. Not only does this provide for ease of transfer to patients, but portable computers may be run on batteries and so, in this way, the computer 36 may be isolated from the mains supply.

The lead box 34 and the computer 36, including the computer display screen and recording and display software for the raw composite data (as opposed to processed data which is specific to this application), as well as their connecting lead are all part of the portable EEG system. The leads 32a, b, c, d, e and their connectors to the lead box 34 and to the electrodes G, Rs, RI, 1, 2, 3, etc. are purpose-built for use in this invention. It is to be noted that a commercial EEG machine proved convenient for use in constructing a prototype apparatus. It is envisaged that bespoke equipment will ultimately prove more suitable for implementing this invention.

The electrodes G, Rs, RI, 1, 2, 3, etc. are commercially available, disposable, self-adhesive neurology electrodes (type 710 01-K) manufactured by Neuroline®. The principal preferences for the electrodes G, R, 1, 2, 3, 4 are that they are low-noise and of a type that is readily attached to a patient in such a way as to result in an impedance at the skin of less than 2kΩ. Moreover they must be of sufficient number to allow effective signal separation by the processing software. Each electrode G, R, 1, 2, 3, 4 with its respective screened lead 32a, b, c, d contributes a single, separate, channel of data to a multichannel recording.

Note that the 710 01-K electrodes sold commercially have a 10 cm length of ordinary (unscreened) cable attached to them. This type was selected as the attached cable length is the shortest available. It is preferred that this length of wire is nearer 1 cm, or that the electrodes are attached directly to the screened leads 32a, b, c, d, e as this would reduce electrical noise further. Disposable electrodes with shorter leads specific to fECG could be trivially made to the same design.

The screened leads 32a, b, c, d are made from 0.9 mm coaxial screened cable of a type suitable for biomedical applications. They should be screened sufficiently to reduce the noise level during fECG recordings to less than 3μV. Such cables make simple, convenient connections from the disposable electrodes G, Rs, RI, 1, 2, 3 to the lead box 34. Connection is made to the lead box 34 by means of a metal D-type connector (not shown) with its body connected to ground, which arrangement provides electrical screening.

The precise number of electrodes G, Rs, RI 1, 2, 3 and respective leads 32a, b, c, d is not important to signal separation, although it does determine the number of distinct sources which will be obtained by the analysis. Electrodes are to be distinguished however by function. That is, the system includes one earth electrode G and two common reference electrodes Rs, RI and a number of electrodes 1, 2, 3 for attachment to the mother's abdomen. As a rough guide, eight or more abdominal electrodes 1, 2, 3 are generally sufficient to provide adequate abdominal coverage and to permit signal separation into sufficient distinct sources. For example, two or three sources generally result from the maternal heart and typically two per fetus. Additional electrodes allow the separation of unwanted artefacts such as those associated with maternal breathing, unwanted electrical interference, etc. Larger numbers of abdominal electrodes 1, 2, 3 may be used, subject only to limits of practicality such as time needed to apply them, comfort and convenience of the mother and limitations of the processing and display systems. Only one common reference electrode Rs, RI is used at a time. One of them RI is connected to a longer screened lead 32f than the other electrodes G, Rs, 1, 2, 3. Thus either a long- RI or short- Rs leaded reference electrode can be placed on the mother, whichever is the more able to reach a conveniently-chosen reference attachment. For example, if the convenient attachment is the mother's ankle, some distance away from the abdomen, the long-leaded electrode RI is used.

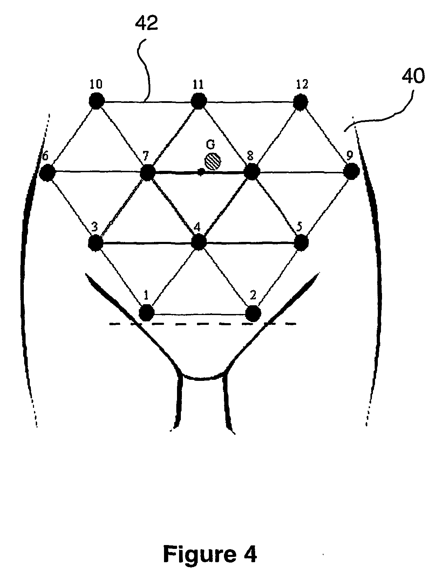

Figure 4 is an illustration of one possible arrangement of electrodes 1, 2, 3, G around the mother's abdomen. In this example, the embodiment comprises twelve abdominal electrodes (1 - 12), the earth electrode G and the common reference electrode Rs or RI (not shown in this figure) which are all attached to the mother's skin 40. Placement is indicated in the Figure by shaded circles indexed with reference numerals of the corresponding electrodes. The common reference electrode RI is attached to the mother's ankle (not shown), the remainder to her abdominal area. Alternatively, the common reference electrode Rs is attached to the mother's abdomen adjacent to G and the remainder also to her abdominal area. Electrode positions 1 - 12 are shown linked by network lines 42 which indicate that an approximately hexagonal arrangement of electrodes is ideally employed for even abdominal coverage. This is not however critical: the degree of separation achieved is not critically dependent on exact electrode location.

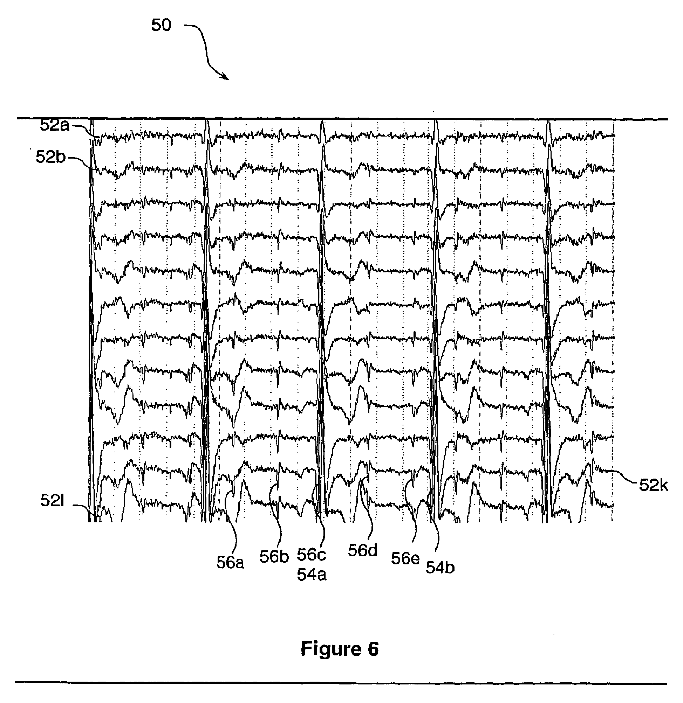

In order to achieve good separation the abdominal electrodes 1-12 should not be placed too close together and should involve a wide coverage of the abdomen. Typically, a regularly spaced arrangement of 12 electrodes, results in an electrode separation of about 10 cm. A practical placement, as shown in Figure 6, includes coverage from one side of the abdomen to, the other and from the pubic hairline to the likely upper limit of the uterus. This latter can be judged from gestation or by following a standard configuration which is sufficient for the maximum height of the uterus (fundal height) which occurs late in pregnancy. It is a feature of this invention that suitable placement can readily be achieved by, for example, a midwife.

The common reference electrode Rs, RI is selected to be of appropriate length for placement at a conveniently-reached point on the mother's body. In some instances the ankle may be appropriate as this is far from the abdomen and the signal that the remaining electrodes 1-12 are detecting. That is, neither signal nor noise will appear artificially reduced when measuring a unipolar voltage difference between abdominal electrode 1 - 12 and reference R.

On the other hand it has been found that placement of the reference electrode Rs on the abdomen has advantages in reducing the amount of noise seen on the screen in the raw composite data. That is, use of an abdominal reference allows use of the short connecting lead 32e. The disadvantage of using a distant location for the reference electrode R is that it necessitates using the long lead 32f. This creates a larger conducting loop, which leads to higher magnetic induction and greater scope for electrical noise to enter the system.

It may therefore prove appropriate to use either reference electrode Rs, RI, depending on the situation. Both options are therefore made available in this embodiment of the invention. It is also possible to employ a combination of reference electrodes Rs, RI and leads 32e, 32f (including additional electrodes and leads as required), such as is used in conventional ECG. Whatever combination is used, the fact remains that all leads 32a, b, c, d, e, f should be as close to the skin and to each other as possible in order to reduce electrical and magnetic noise through magnetic flux linkage of loops formed by the combination of mother and leads.

The earth electrode is placed in position G, close to the mother's navel. Again, an alternative site close to the abdominal area may be chosen.

In preparation for attachment and recording, the mother will ideally lie comfortably on a bed with the lead box 34 close by, but touching neither the patient nor the bed frame. She should be allowed to relax for a few minutes to help reduce involuntary muscle activity.

The screened leads 32a, b, c, d connect the electrodes 1 - 12, G, R to the lead box 34. An outer braided mesh layer of the coaxial cable comprising each screened lead 32a, b, c, d is connected to isolated ground at the lead box 34 and to the metal case of the D-type connector. The earth electrode G is also connected to isolated ground at the lead box 34. This provides a return bias current path to the mother's body for common mode interference which will not be passed by the amplifier 38.

Voltage signals arising from cardiac activity and other sources are picked up by the electrodes 1 - 12, R attached to the skin 40. The signals are then communicated to the lead box 34 via the screened leads 32a, b, c, d. The lead box 34 is the SAM 25R "headbox" of the Micromed Electronics EEG system. The advantage of an EEG headbox as opposed to an ECG lead box, is that the former has superior electronics (accordingly less noisy electronics) and an increased number of input channels available for use. The input channels are, importantly, configured for unipolar use.

The particular lead box 34 used has a number of possible connections (more, in fact, than are required in order to implement the present invention). There are 21 unipolar input channels and 5 ground connections in addition to the common reference connections. Further specifications of the SAM 25R lead box of relevance to a prototype apparatus built to implement this invention are: touch-proof safety connections, 512 Hz sampling, passband from 0.5 - 256 Hz, low-pass anti-aliasing filter with cutoff frequency at 1 kHz and 12-bit resolution covering a voltage range of $\pm$ 2mV.

Clearly in connecting the electrode arrangement shown in Figure 4 to the lead box 34 only 12 of the 21 unipolar channels are used. Additional abdominal electrodes may therefore be used if required. This may be a useful facility in special cases (for example triplets or higher multiple pregnancies) in which there is particular concern for the health of the fetus or fetuses.

The multiple channel inputs to the lead box 34 are used in a unipolar configuration. That is, voltage readings are taken between each abdominal electrode 1 -12 and the common reference electrode R (whichever Rs, RI is selected).

This is to be compared with prior art ECG devices which have attempted to solve the problem of system noise by taking bipolar readings.

A conventional ECG reading is taken between electrodes arranged on a patient's chest and a specifically configured reference formed from leads located on the patient's wrists and ankles. In this way six unipolar readings are available for processing. Adaptation of conventional ECG equipment to measure fECGs by positioning ECG electrodes on the mother's abdomen encounters two fundamental problems. The first is that the noise level within the equipment itself is too high. The second is that only six channels are available. Although in some instances, for example a singleton pregnancy some way into gestation, this may be sufficient to separate the fetal signal, there may be insufficient coverage for more complicated situations requiring data separation using an ICA algorithm. For a "blind" approach, that is one in which no knowledge of fetal position or presentation is assumed *a priori,* it is recommended that more than six electrodes, and more preferably eight, are used to achieve appropriate coverage.

The bipolar procedure was adopted by Callaerts when attempting to derive fECG details from measurements taken at the mother's abdomen. Use of such pairs of electrodes and the SVD algorithm renders the measurements highly dependent on the geometric arrangement of electrodes and orientation of the fetus in the uterus.

The unipolar configuration has become a practical proposition in the present invention by the careful reduction of electrical noise. This is why use has been made of the low noise electrical electrodes, screened leads and electronics of the EEG headbox. This therefore overturns the long-held belief that such an arrangement would be incapable of overcoming the noise problem.

Within the lead box 34, analogue voltage signals from each abdominal electrode (1 - 12) are fed to one input of a respective differential amplifier 37 and the voltage signal from the reference electrode (R) is fed to the other. Each differential amplifier 37 therefore outputs an amplified signal proportional to the difference between the voltage developed at the associated abdominal electrode (1-12) and that developed at the reference electrode (R): a unipolar voltage. The resulting amplified signals are filtered by respective anti-aliasing low-pass filters 38 and digitised by the simultaneous multi-channel A/D converter 39. The advantage of using a multi-channel A/D converter 39 is that simultaneous sampling can be arranged on all channels (1 -12). These digitised signals are then passed to the computer 36 for signal processing.

It is to be noted that although a unipolar configuration has advantages, a bipolar configuration is by no means precluded. One may be replicated simply by taking differences between digitised unipolar channel outputs, if such a bipolar configuration is required.

In setting up the equipment for making ECG and fECG recordings it is important to reduce ambient and system noise. The following procedure has been found to produce sufficiently low-noise readings:

i). The mother's skin 40 where each electrode 1 - 12, G, R is to be placed is lightly excoriated using standard abrasive preparation tape (e.g. "Skinprep", manufactured by. 3M) and then cleaned with an alcohol- or water-based swab.

ii). Each electrode 1 - 12, G, R, a 2 cm self-adhesive pad, is attached to the skin with light finger pressure, and arranged such that the short trailing wire points towards the earth electrode G.

iii). Each electrode 1 - 12, G, R is then connected to the corresponding screened lead 32a, b, c, d.

iv). The screened leads 32a, b, c, d are connected to the lead box 34 via the D-type screened connector (not shown).

v). The recording system is switched on, using battery power only - i.e. isolated from the mains supply.

vi). The skin impedance at each electrode is measured and any electrode having a skin impedance greater than about 2kΩ is reapplied.

vii). Individual screened leads are gathered together and, in addition, held as close to the mother's skin 40 as possible in order to minimise magnetic pickup.

viii). The recording system (electrodes 1 - 12, G, R, leads 32a, b, c, d and lead box 34) is set to display real-time signals from abdominal electrodes 1 - 12. On screen traces corresponding to outputs from all electrode channels are displayed simultaneously.

ix). Possible sources of electrical interference (such as mains leads in the room) are disconnected if possible.

x). The mother is asked to relax as much as possible and her posture is adjusted (for example, using pillows under her legs, ankles, etc.) until the noise level of all the traces is less than 10 μV and preferably as low as possible.

xi). Once the operator is satisfied as to the quality of the traces, recording is started. The computer 36 records composite raw data represented in the traces and any display settings and saves them to, for example, its hard disk.

These various steps contribute to lowering the noise level as far as possible. Another surprising observation has been made following the procedure listed at step x). This is, that maternal muscle noise is a major contributing factor to the noise of the system. Once steps such as ensuring that the mother is sufficiently relaxed are taken, the overall noise can be made to fall significantly. This, plus the electrical noise reduction described above, has been found in this embodiment of the invention to reduce noise sufficiently to allow use of unipolar channel inputs. It has previously been unappreciated that muscle noise made such a significant contribution.

Once sufficient data has been collected, recording is stopped, the screened leads disconnected from the lead box 34 using the D-type connector and the electrodes 1 - 12, G, R removed from the mother.

Using prototype apparatus, it has been found that each recording takes approximately 15 minutes, including application and removal of the sensors.

There are two major advantages of the hardware described in relation to this embodiment of the invention. The first is that commercial-off-the-shelf, portable, battery-powered equipment is used. This makes the system relatively inexpensive and also mobile. Recordings can be taken at home, by a hospital bed, etc wherever convenient. The second advantage is that the same equipment is capable of performing neo-natal and adult ECGs. Alternative processing of the signals will be required: that is, six channel unipolar inputs will have to be processed in order to generate a conventional 12-lead ECG trace and additional reference connections (ECG requires that the reference used is an average signal from a set of reference voltages measured at standard locations), for example to the subject's wrists and ankles, will be required.. However this is a straightforward matter, and the approach to take will be readily apparent to one skilled in the art. The ability to take neo-natal measurements is important because it enables a better comparison between fECG and neo-natal ECG to be made. Equipment differences may blur the comparison if traces are obtained using separate pieces of apparatus. Prior art fECG monitors are made from specialised hardware which is not capable of being adapted to take conventional ECGs.

As mentioned previously, the SAM 25R EEG "headbox" 34 is described in relation to this embodiment as it was the most appropriate for use in a prototype. That is, it was readily adaptable to perform the functions required and so avoided the need to build bespoke equipment at this stage of development. It is of course to be expected that improved performance can be obtained with a purpose-built lead box 34. An improved design would include features which match the performance of the lead box 34 more closely with the fECG data. In particular, the SAM 25R lead box 34 has a non-ideal low-pass filter at its input and an amplifier which is overly noisy. It is anticipated that the filter should be redesigned with a passband that rejects frequencies greater than around 200 Hz, as opposed to the ~1 kHz limit of the EEG headbox. This feature would reject more unwanted noise and provide improved anti-aliasing. Amplifiers with noise less than 0.1 $\mu$V are available and should preferably be used (the EEG box amplifier has a noise level of 0.16 $\mu$V). Such a redesign would improve acquisition of P and T waves in the fetal heart-beat complex, which can be of just 1 $\mu$V or less in amplitude. Additional connections, to allow for the additional reference limb connection for conventional ECG, should also be provided.

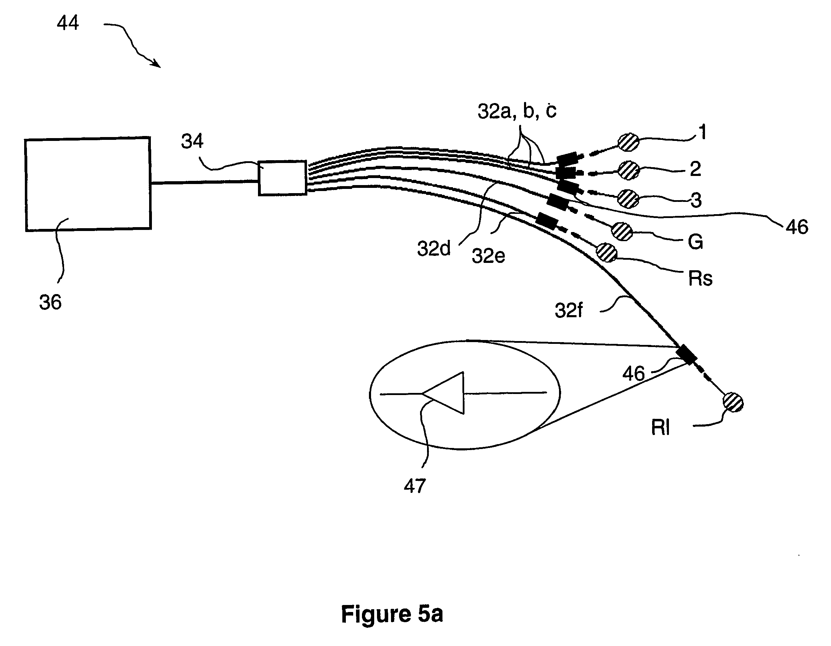

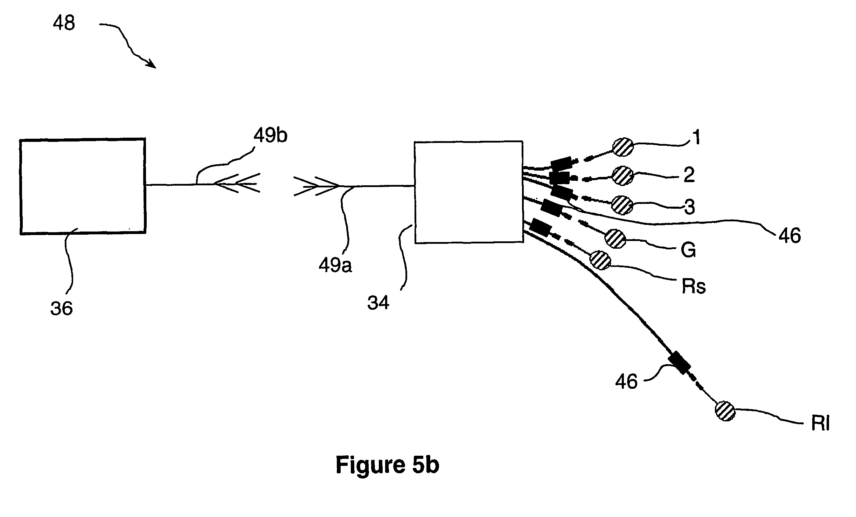

Alternative embodiments of apparatus suitable for implementing this invention are shown in Figures 5a and 5b. In both these Figures, components of the system(s) common to those shown in Figure 3 are like referenced.

**Considering Figure 5a first,** this embodiment 44, as with the earlier prototype 30, comprises a number of electrodes (G, Rs, Rl, 1, 2, 3, ...) suitable for attaching to the mother's skin and monitoring voltages signals generated thereon. In this embodiment however, each electrode (G, Rs, Rl, 1, 2, 3, ...) is connected first to its own pre-amplifier 46 (illustrated schematically inset 47) and from there, via respective screened leads (32a, b, c, d, ....), to the lead box 34. The detail of electronics suitable for preamplification will be apparent to one skilled in the art. As before, the lead box 34 contains the differential amplifiers 37 and low-pass filters 38 for each electrode channel and the multi-channel A/D converter 39. The computer 36 performs data processing on the digitised output from the lead box 34.

In the Figure 3 embodiment 30, the lead box 34 houses the electronics responsible for performing all processing functions for the different input channels (via leads 32a, b, c, d, .....). These functions include:

amplification, low-pass anti-alias filtering, high-pass filtering, digitisation and optical isolation. In this present embodiment 44, individual amplifiers 47 are disposed adjacent respective electrodes (G, Rs, Rl, 1, 2, 3, ...) and each amplifier 47 therefore provides a stage of pre-processing on its individual channel. In this way, the signals propagating along the leads 32a, b, c, d, ... have already been amplified to some extent and are therefore much larger and more robust to electric and magnetic noise sources. As a consequence electric and magnetic noise in the leads is far less significant than for the embodiment 30 shown in Figure 3.

An electric guard potential may be applied to the earth shielding on the screened leads 32a, b, c, d, ... Methods of implementing this will be apparent to one skilled in the art. The guard potential has the effect of reducing lead capacitance and minimising mismatch between input capacitances. This increases the common mode noise component of the detected signal that is rejected by the differential amplifier 37. Although the guard potential may be similar to the signal voltages of interest, the earth shielding must be driven from a low impedance source. For example, from a voltage follower driven by the signal of interest.

Figure 5b illustrates a further embodiment 48 of the present invention. This embodiment 48 also comprises a number of electrodes (G, Rs, RI, 1, 2, 3, ...) suitable for attaching to the mother's skin and monitoring voltage signals generated thereon, with each electrode (G, Rs, RI, 1, 2, 3, ...) being connected to its own dedicated pre-amplifier 46. In this embodiment 48 the lead box 34, although housing the same electronics as described in relation to previous embodiments 30, 44, is also connected to a transmitter 49a. A corresponding receiver 49b is connected to the computer 36. Preamplification is again performed at the electrodes, as for the embodiment 44 shown in Figure 5a. The lead box 34 is however placed close to or on the patient (for example, using a belt) and its output is transmitted to the computer 36 over a wireless (for example, infra red) link 49a,b. Transmitting amplified data in this way enables the screened leads 32a, b, c, d, ... to be far shorter than previously: they only need reach the closely-positioned lead box 34. This further reduces the amount of noise and signal loss arising from the leads 32a, b, c, d, ... In addition, the lack of long trailing leads and their physical connection to the computer 36 enables the mother to move around more freely, without leads or electrodes having to be disconnected, potentially allowing her to relax more readily when a recording is to be taken. Finally, this embodiment also offers the potential for readings to be made whilst the mother is ambulatory, if sufficient relaxation can be induced.

Figure 6 is an illustration 50 of 5s worth of raw composite data traces taken using the equipment described in Figure 3 attached as shown in Figure 4. Twelve traces 52a, b, ...., l are generated corresponding to the twelve abdominal electrodes 1 - 12 of Figures 3 and 4. In all traces both maternal 54 and fetal 56 heart-beats are visible. For example, trace 52k exhibits a number of fetal heart-beats ..... 56a, 56b, 56c, 56d, 56e......, although one of these 56c is masked by the far stronger maternal signal 54a. However it can readily be seen that information relating to the detail of the fECG in particular is not at all apparent.

Referring once again to the embodiment 30 shown in Figure 3, the computer 36 receives, from the lead box 34, digital data relating to the traces 52a, b, c ....... l for signal processing. In this embodiment of the invention the digitised signals are filtered (this time in software) in order to remove further unwanted frequency components. The filters used consist of a high-pass infinite impulse-response (IIR) filter of 6 filter taps, and a low-pass finite impulse-response (FIR) filter of 9 filter taps. The high-pass filter is designed using an IIR Butterworth filter with a passband of 2Hz, stop-band of 0.1 Hz and stop-band attenuation of 120dB, resulting in a 3dB point of 1 Hz and a passband ripple of 0.01 dB. The low-pass filter is designed using a Blackman window with a band-edge at 150Hz. Filtering is implemented using a zero phase forward and reverse digital filtering technique. This band-pass filtering reduces baseline wander to acceptable levels and also removes high frequency interference which lies outside the frequency range of interest.

The filtered signals are then subject to a Blind Signal Separation (BSS) technique based on Independent Component Analysis (ICA), I.J.Clarke "Direct Exploitation of non-Gaussianity as a Discriminant", EUSIPCO '98, Rhodes, Greece, 8 - 11 September, 1998. ICA is a powerful statistical and computational technique for revealing hidden factors that underlie sets of random variables, measurements or signals. In this situation therefore it is used to analyse the twelve signal traces 52a - l obtained from the abdominal electrodes 1 -12. ICA defines a model for observed composite data variables $\underline{x}_i$ based on the assumption that each is a linear or nonlinear mixture of some unknown latent sources $\underline{s}_j$. The mixing system is also unknown and the sources are assumed mutually independent and non-Gaussian. In cases for which the composite data variables are provided as a set of parallel signals or time series, and no prior knowledge about the signals, sensors or method of propagation, etc. is employed, the term Blind Source Separation is used to characterise the problem.

Thus, in this instance, the electrodes 52a - l will be considered indexed by the subscript $i$, each of the $i = 1,...,n$, electrodes producing a sensor output $\underline{x}_i$. Each sensor output $\underline{x}_i$ has been digitised by the lead box 34 and so comprises m time samples of recorded data. The ICA algorithm takes the $m \times n$ matrix $X$ of sensor outputs and generates a mixing matrix M and a set of n independent sources $s_j$ such that each sensor output $x_i$ can be written as a different linear combination of the sources $s_j$ i.e.:

$$\underline{x}_i = \sum_{j=1}^{n} m_{ij} \underline{s}_j \quad \text{or simply} \quad X = SM \quad (1)$$

where X is a matrix whose columns are the n sensor outputs $\underline{x}_i$ and S is the m x n matrix whose columns are the set of n independent sources $\underline{s}_j$.

In this way the composite data X is separated into different independent sources of interest $\underline{s}_j$. The various sources will comprise the fetal ECG, maternal ECG and also some separated unwanted noise sources. In multiple pregnancies there will, of course, be more than one fetal ECG. Signals of interest relating to selected sources may be separated and examined individually in isolation.

This model assumes that the sources $\underline{s}_j$ are point sources, which is clearly not the case for a physiological source such as the heart which is of finite extent. In this situation it is an artefact of the calculation that multiple, separated sources are found. By using abdominal sensors alone the number of sources found per heart varies from about one to three but depends on factors such as proximity of the electrode to the source, fetal presentation and details of electrical conduction to the surface. This then is the origin of the requirement of two electrodes per fetal heart which was referred to previously.

A beneficial additional consequence is the ability to observe the variation in the structural morphology of the fetal heart-beat over the maternal abdomen. In a standard ECG it is well known that the detail (morphology) of the measured heart-beat waveform varies over the chest i.e. depending on which electrode the signal is observed. For example, the P wave is known to appear biphasic, rather than the peak shown in Figure 2, if observed at certain chest positions. This variation in heart-beat morphology may also be observed in the fECG over the mother's abdomen using the equipment described herein. This provides a confirmation to a doctor that the technique is functioning as intended, as well as perhaps indicating fetal presentation and position and may ultimately prove to be an additional diagnostic tool.

ICA is a well known analytical technique and it is not necessary to expand on the presentation given herein. Further details are described in "Independent Component Analysis - theory and applications" by T-W. Lee, published by Kluwer Academic, Boston (1998). Many developments of the basic ICA technique are also well known and it is anticipated that these may well be applied beneficially to the signal processing described herein. In particular, signal separation should be achievable in real time, and run as the data is recorded. Other improved algorithms, tailored to the specific application, should enable application of the fECG technique described herein to situations in which continuous monitoring is required. Such monitoring is known to be important to a number of areas, including long-term assessment of heart-rate variability, identification of intermittent cardiac arrhythmias and anomalies and the use of fECGs during labour.

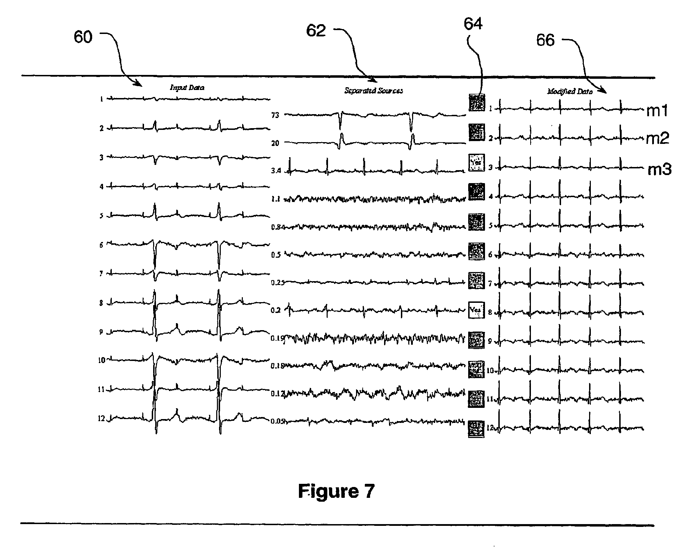

Figure 7 illustrates stages involved and results obtained in processing data collected from a singleton pregnancy using the apparatus described herein. The Figure is divided into three columns 60, 62, 66. The first column 60 comprises 12 channels of input data, one channel collected at each electrode, the number of the electrode being indicated at the left hand side of each trace. Accordingly, this column contains a portion of the information shown in Figure 6. A second column 62 illustrates the 12 separated sources found by ICA. Each source is annotated at its left hand side with the percentage of total energy found in that source, and reference will be made to this value when referring to individual sources. To the right of each source is a source-selection button 64. A third column 66 comprises modified data generated using only those sources from the second column 62 which are of interest.

Examination of the separated sources 62 readily indicates that two sources have been found for the maternal heart-beat: the two strongest sources 73, 20 show pulses of the expected frequency. Similarly two weaker sources 3.4, 0.2 exhibit readily noticeable pulses at a typical fetal heart-beat frequency and are aligned with each other. The remainder of the sources typically correspond to noise sources such as maternal breathing, muscle noise, mains and other electrical interference, etc.

In this example it is assumed that the fetal ECG is of interest. In order to extract this, each electrode trace 1-12 in the first column should be reconstructed using only the fetal heart sources 3.4, 0.2. That is, each electrode trace 1-12 is first modelled as a mixture of sources 73 - 0.05; and then reconstructed using only those sources, and associated coefficients, of interest ($x_i(m) = \Sigma_j m_{ij}s_j$, $j$ being restricted to the index associated with particular separated sources). This gives rise to twelve modified data traces m1 - m12, as illustrated in the third column 66. In these traces m1 - m12, the fetal heart-beat is readily apparent and the maternal and other noise sources have been suppressed.

In this embodiment of the invention, selection of the required sources is made by means of the source-selection button 64 which is displayed on the computer 36 next to each separated source 73 - 0.05. Each button may be toggled between a "no" indicator, meaning discard the source and a "yes" indicator, meaning to make use of it. Selection may be made by the operator. Figure 6 illustrates that the fetal sources 3.4, 0.2 are selected by means of the source-selection button 64. It is clear however that this selection process can readily be automated, thus enabling a fetal trace to be displayed without the need for operator intervention.

From this analysis it can be seen that, although noise reduction is important in enabling a fECG to be extracted from the raw composite data, an apparently noisy signal may still prove tractable. This is because, as can be seen, separable noise will be isolated by the ICA technique and can be discarded. The problem is that separable noise is not often distinguishable from unseparable noise in the composite signal. Consequently, all possible noise is minimised in constructing and operating apparatus in accordance with this invention. This gives the best chance for extraction of the fECG, although clearly it may still be extractable from an apparently noisy signal under certain circumstances.

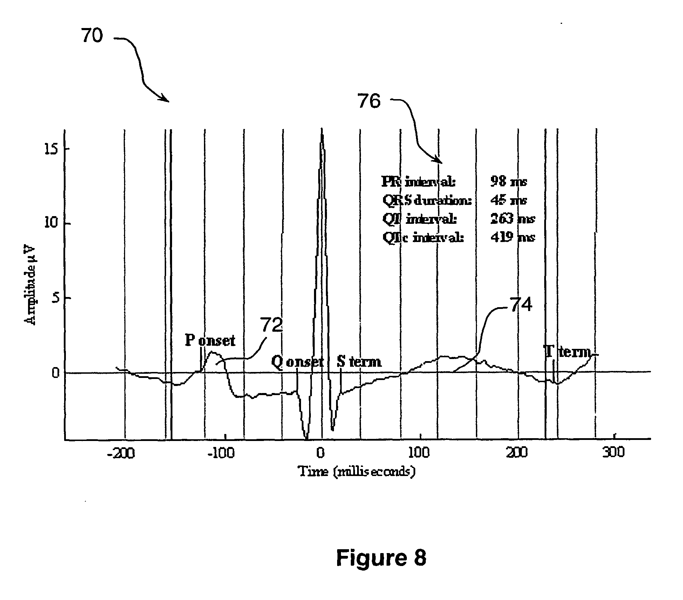

Figure 8 illustrates a representative average 70 of the underlying waveform of the modified data m1 - m12 shown in the third column 66 of Figure 7. This data is extracted in accordance with standard methods of averaging the signals in the area around the peaks in a particular trace. The P wave 72 and T wave 74 are clearly seen in addition to the QRS complex. Positions of P and Q wave onsets and S and T wave terminations are also marked. From these details, information 76 concerning certain diagnostically important intervals has been extracted. The QT interval is a particularly important diagnostic parameter but it depends on determination of the end of the T wave. No prior art example of fECG has managed to quantify this parameter. It is clearly displayed as 263 ms in the display of Figure 8.

In the present embodiment of the invention determination of the positions of wave onsets and terminations and performance of consequent calculations of intervals are carried out semi-automatically. That is, cross-hairs are displayable on the display screen and can be electronically dragged and dropped by an operator. Once marker positions have been set in this way, the diagnostic intervals are automatically calculated by the computer 36 and added to the display and to an automatically generated patient record. It is a straightforward matter for one skilled in the art to provide for fully automatic characterisation and measurement of fetal waveforms.

Underlying waveforms 70 such as that shown in Figure 8 may be generated for each individual electrode 1 - 12 channel and displayed superimposed in an outline of the abdominal surface. This abdominal surface map therefore shows the average fetal waveform at each electrode position 1 - 12 and this can be used by a doctor to assess fetal health and development. The waveform 70 shows the expected morphological variation, which is expected to be of assistance in clinical diagnosis.

Abdominal surface intensity maps are readily generated from the separated sources by shading a map of the geometrical locations of the sensors according to the strength of the coefficient $m_{ij}^2$ of the desired source $j$ (or combination of sources) at each sensor $i$. Brighter areas are used to indicate higher levels of signal strength, and the different fetal positions can be distinguished in a multiple pregnancy.

In using this equipment, waveforms 70 may be extracted for each fetal heart-beat. Not only is the waveform 70 a useful diagnostic parameter itself, but it may also be used for "gating" another imaging tool such as ultrasound. The location of the peak in the activity of the fetal heart-beat is recorded. This location is fixed on the cardiac cycle and is obviously useful in determining the instantaneous heart-rate of the fetus. It can also be used however to send a pulse at the appropriate time to a second piece of monitoring or imaging equipment such as one based on Doppler ultrasound. Imaging can be improved by making use of information about the position of the cardiac cycle. For example, 3D images of the fetal heart may be reconstructed from time-aligned 2D ultrasound images. Timing information from the fECG peak locations can therefore be used to reduce motion artefacts.

Signal separation does not have to be carried out using ICA, but this technique is very much preferred. SVD, another commonly-used analysis tool in signal processing, requires, for fECG separation, careful consideration of the geometrical relationship between the abdominal electrodes. With the reduction in noise provided by the hardware of this invention, it is envisaged that less sophisticated signal processing techniques may also yield acceptable results. For example, if an additional electrode is placed on the mother's heart, the maternal signal can be subtracted from the composite signals simply by removing components which correlate with the additional electrode signal. Of course the noise contributions would not be separated out, but if these are sufficiently low, useable results may be obtained. It is a feature of this invention that alternative or further improved signal separation techniques may be incorporated primarily with software changes and without substantial alteration to the equipment.

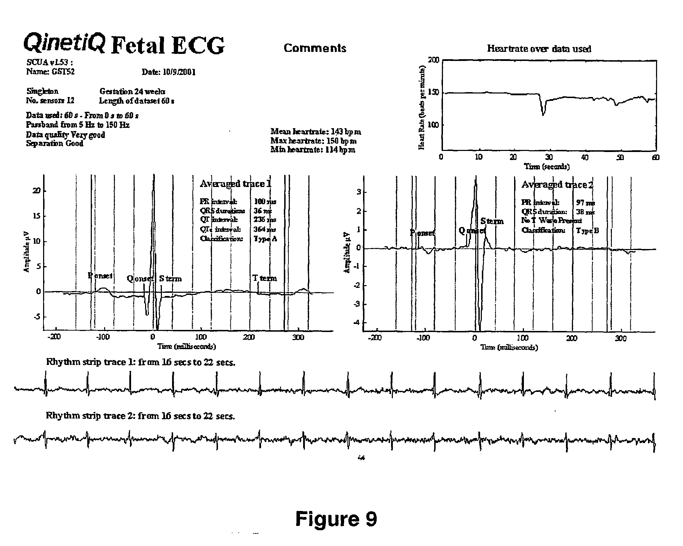

Use of a computer 36 to display the processed data clearly offers extreme flexibility in which of the range of extractable parameters is displayed. An example display in shown in Figure 9.

It is clear that a variety of parameters may be set up for display and output. Examples of apparatus capabilities which it is envisaged will prove useful are:

i). Display of patient data, recording and processing details for patient and hospital records.

ii). Display of the multichannel abdominal input data (unipolar or chosen bipolar configuration).

iii). Means for manual or automatic selection of the source or sources of interest e.g. fetal, maternal, or raw data channel in the case of the use of the equipment for performing conventional ECG.

iv). Ability to perform a projection of any or all of the channels of data onto the subspace spanned by the selected sources i.e. to eliminate the contributions to all the data channels from sources other than the source or sources of interest, and display the results.

v). Detection of the positions of the QRS peaks to be used as fiducial markers for the time-sequenced averaging of the cardiac waveform. A number of different peak detection algorithms can be employed including the use of a simple threshold.

vi). Plotting and reporting of heart rate over the recording interval together with statistical parameters concerning the heart rate and its variability. These parameters include: maximum, minimum and mean heart rate, presence of gross changes in heart rate, maximum variation in heart rate (maximum - minimum heart rate), relative variation in heart rate (maximum variation divided by mean heart rate), coefficient of variation (standard deviation divided by mean heart rate), etc.

vii). Fitting of data window around the marker for averaging purposes.

viii). Averaging of the data windows time-aligned to the marker to produce an average waveform for the chosen data channel.

ix). Parameterisation of the average waveform by manual or automatic labelling of the positions of such features as the P-wave onset, the Q-wave onset, the S-wave termination and T-wave termination in order that the PR interval, QRS duration and QT interval and any other parameters of interest may readily be determined.

x). Automatic generation and annotation of parameters such as PR and QT intervals and QRS duration from the semi-automatic measurements of P-wave and Q-wave onsets and S and T- wave termination on a screen displaying the average fetal waveform.

xi). Option to display a rhythm strip of a standard length on the screen or of particular section of interest.

xii). Option to display a full set of rhythm strips such as one minute on one page.

xiii). Option to enter and display patient details on screen.

xiv). Automatic generation of a patient record containing all the parameters of interest along with the average fECG waveform, the fetal heart rate over the interval of data of interest and the chosen rhythm strip which might include particular abnormalities or features of interest.

xv). Automatic appending of results to a database of previously generated patient data.

xvi). Abdominal surface map of features of interest such as source strength over the abdomen, or average fECG waveform at the positions of the electrodes.

xvii). Ability to zoom in or out of the display in order to focus on fine detail in any view for detailed analysis of features such as the structure of the heart-beat, heart rate or waveform.

In addition to monitoring the fECG during pregnancy, the apparatus described herein may also be used during labour. This may appear somewhat at odds with the requirement for the mother to be relaxed, but very useful measurements can be extracted at certain times, such as the time following a contraction. Whilst maternal muscle noise and uterine contractions will add to the background noise level, there are at least two mitigating factors that make the problem less intractable. First, high-risk pregnancies are often delivered under epidural anaesthetic. Under these conditions the mother's movements are limited. Secondly, it is the variation in the fetal heart activity in response to (i.e. soon after) a uterine contraction that is of particular clinical interest. Accordingly, it is not essential (although it may be desirable) for monitoring to continue throughout contractions. To this end it is envisaged that the signal processing technique may be varied so as to make use of information gained between contractions in order to follow the fetal heart activity through the next contraction.

It has been shown by invasive fECG using a fetal scalp electrode that analysis of the ST segment of the heart-beat in particular has diagnostic value during labour for the identification of hypoxia and fetal distress. Use of apparatus according to this invention in the manner described above will enable this information to be obtained non-invasively. The display should also be altered in order to present information useful during labour such as, for example, a continuous plot of the fetal heart activity alongside the maternal heart activity and uterine contractions in real time. Real-time signal separation may be achieved, for example, by consecutive processing of a number of overlapping blocks of data. The outputs from this processing can then be aligned to produce a continuous separated fetal signal using correlation of the output of consecutive blocks of data.

**Claims**

1. Apparatus for the detection of a fetal electrocardiogram (fECG), the apparatus (30, 44, 48) comprising a plurality of electrodes (1-3, R) for application to a patient and signal processing means (34, 36, 46, 47) for deriving an fECG from signals developed by the electrodes (1-3, R), **characterised in that**:

   a) the electrodes are low-noise electrodes (1-3, R) for abdominal skin placement during pregnancy and have associated leads with screening to reduce noise level and being in number sufficient to enable at least six signal sources to be monitored; and

   b) the signal processing means is a low-noise signal processing means (34, 36, 46, 47), is connectable to the electrodes (1-3, R), has sufficient sensitivity to detect signals of magnitude comparable with electroencephalography signals, and is arranged to:

      i) detect signals developed by the electrodes; and

      ii) process electrode signals to obtain data and use a Blind Source Separation technique to process the data and distinguish independent source contributions thereto and derive at least one source signal (3.4, 0.2) relating to a single fetus fECG.

2. Apparatus according to claim 1 **characterised in that** the electrodes (1-3, R) are sufficient in number to enable at least eight signal sources to be monitored.

3. Apparatus according to claim 1 or 2 **characterised in that** the signal processing means is arranged:

   a) to record signals indicative of voltages developed between pairs of electrodes (1-3, R) in a plurality of signal channels (60);
   b) to process digitised data derived from electrode voltages in order to generate at least one source signal (3.4, 0.2) which relates to the fECG of a single fetus; and
   c) on identification of the at least one source signal (3.4, 0.2) relating to the single-fetus fECG, to reconstruct, for at least one signal channel, that component of the digitised data within the channel which is attributable to that fetus, and which therefore corresponds to a single-channel fECG.

4. Apparatus according to claim 1, 2 or 3 wherein the signal processing means (34, 36, 46, 47) is arranged to process the data in digitised form to generate a plurality of separated source signals (62), at least one of which (3.4, 0.2) relates to the fECG of a single fetus.

5. Apparatus according to any one of claims 1 to 4 **characterised in that** the electrodes (1-3, R) are placeable on the skin such that skin impedance at each electrode is less than 5kΩ.

6. Apparatus according to claim 5 **characterised in that** the electrodes. (1-3, R) are placeable such that the skin impedance at each electrode is less than 2kΩ.

7. Apparatus according to any one of claims 1 to 6 **characterised in that** the electrodes comprise a plurality of abdominal electrodes (1-3) for placement on the skin (40) in the abdominal area and a common reference electrode (R), and the signal processing means (34, 36, 46, 47) is arranged to record signals indicative of voltages developed between each abdominal electrode (1-3) and the reference electrode (R).

8. Apparatus according to claim 7 **characterised in that** the electrodes also include a low-noise electrode (G) which is connected to earth when the apparatus is in use.

9. Apparatus according to claim 7 or 8 **characterised in that** the signal processing means (34, 36, 46, 47) comprises electronic components (34, 46, 47) for processing analogue voltage signals developed between pairs of electrodes to provide digital signals in a plurality of signal channels (60) and data processing means (36) to process the digital signals.

10. Apparatus according to claim 9 **characterised in that** the electronic components (34, 46, 47) incorporate a low-noise differential amplifier (37) for amplifying the difference between each electrode's voltage signal and a signal derived from the voltage developed at the reference electrode (R), an anti-aliasing low-pass filter (38) and an analogue to digital converter (39).

11. Apparatus according to claim 10 **characterised in that** the electronic components are located in a multichannel lead box (34) remote from a patient and suitable for electroencephalography (EEG) use, and the electrodes (1-3, R) are connectable to the multichannel lead box (34) by respective screened leads (32a, 32b, 32c, 32e).

12. Apparatus according to claim 10 or 11 **characterised in that** the electronic components (34, 46, 47) additionally include, for each electrode (1-3, R), a respective pre-amplifier (47) located adjacent that electrode (1-3, R) and connectable to the multichannel lead box (34) by a respective screened lead (32a, 32b, 32c, 32e).

13. Apparatus according to claim 10 **characterised in that** the electronic components (34) are located in a lead box (34) for positioning adjacent to a patient, and the lead box (34) and data processing means (36) are arranged to communicate via a wireless link (49a, 49b).

14. Apparatus according to any preceding claim **characterised in that** the signal processing means (34, 36, 46, 47) has signal channels (60) which exhibit a noise component within raw composite data of less than 10μV.

15. Apparatus according to claim 14 **characterised in that** the signal channels (60) exhibit a noise component of less than 5μV and preferably less than 3μV.

16. Apparatus according to any preceding claim **characterised in that** the signal processing means (34, 36, 46, 47) is arranged to identify whether or not a plurality of source signals (3.4, 0.2) relate to a single fetus, and if so to combine such signals with appropriate weighting in a reconstruction of the single-channel fECG.

17. Apparatus according to claim 16 for the detection of fetal electrocardiograms in a multiple pregnancy, **characterised in that** the signal processing means (34, 36, 46, 47) is arranged, on identification of the at least one source signal relating to each fetus, to reconstruct, for at least one abdominal electrode (1-3), components of the digitised data within the corresponding signal channel attributable to each fetus.

18. Apparatus according to claim 17 **characterised in that** the signal processing means (34, 36, 46, 47) is arranged to reconstruct, for each abdominal electrode (1-3), components of the digitised data within each corresponding signal channel which is attributable to each fetus and thereby to construct an abdominal surface intensity map of signal strength from each fetus.

19. Apparatus according to any preceding claim **characterised in that** the signal processing means (34, 36, 46, 47) is arranged to separate source signals by Independent Component Analysis (ICA).

20. Apparatus according to claim 1 **characterised in that** the signal processing means (34, 36, 46, 47) incorporates computer apparatus **(36)** programmed to:

a) filter multi-channel digitised composite signals, each signal corresponding to a difference between a voltage developed at a signal electrode (1-3) and that developed at a reference electrode (R), in order to remove unwanted frequency components;
b) generate a plurality of separated source signals (62) from the corresponding plurality of filtered composite signals each treated as a linear mixture of unknown source signals *(X = SM);*
c) identify, either automatically or by prompting for a user input, a source signal or source signals (3.4, 0.2) corresponding to a single-fetus ECG;
d) reconstruct, for each single-fetus ECG identified, and for at least one signal channel, that component of the filtered composite signal within the channel which is attributable to that fetus and therefore corresponds to a single-channel fECG; and
e) display at least one reconstructed fECG to a user.

**Patentansprüche**

1. Gerät zur Erfassung eines fötalen Elektrokardiogramms (fEKG), wobei das Gerät mehrere Elektroden (1-3, R) zur Anbringung an einem Patienten und eine Signalverarbeitungseinrichtung (34, 36, 46, 47) zur Ableitung eines fEKGs aus Signalen aufweist, die an den Elektroden (1-3, R) auftreten, **dadurch gekennzeichnet, dass**

a) die Elektroden rauscharme Elektroden (1-3, R) zum Aufsetzen auf die Haut des Abdomens während der Schwangerschaft sind, damit verbundene Leitungen mit Abschirmung zur Verringerung des Rauschpegels aufweisen und in einer Anzahl vorliegen, die ausreicht, um mindestens sechs Signalquellen überwachen zu können, und
b) die Signalverarbeitungseinrichtung eine rauscharme Signalverarbeitungseinrichtung (34, 36, 46, 47) ist, mit den Elektroden (1-3, R) verbindbar ist, ausreichende Empfmdlichkeit besitzt, um Signale einer mit elektroenzephalographischen Signalen vergleichbaren Stärke zu erfassen, und so ausgebildet ist, dass sie

i) von den Elektroden gelieferte Signale erfasst und
ii) die Elektrodensignale so verarbeitet, dass Daten erhalten werden, und ein Verfahren zur Blinden Quellentrennung (Blind Source Separation) zur Verarbeitung der Daten und zur Unterscheidung der unabhängigen Quellenbeiträge dazu und zur Ableitung mindestens eines Quellensignals (3.4, 0.2) anwendet, das sich auf ein fEKG eines einzelnen Fötus bezieht.

2. Gerät nach Anspruch 1, **dadurch gekennzeichnet, dass** die Elektroden (1-3, R) in einer Anzahl vorliegen, die ausreicht, um mindestens acht Signalquellen überwachen zu können.

3. Gerät nach Anspruch 1 oder 2, **dadurch gekennzeichnet, dass** die Signalverarbeitungseinrichtung so ausgebildet ist, dass sie

a) Signale aufzeichnet, die Spannungen kennzeichnen, die zwischen zwei Elektroden (1-3, R) in mehreren Signalkanälen (60) auftreten,

b) von den Elektrodenspannungen abgeleitete digitalisierte Daten verarbeitet, um mindestens ein Quellensignal (3.4, 0.2) zu erzeugen, das sich auf das fEKG eines einzelnen Fötus bezieht,

und

c) bei Identifizierung des mindestens einen Quellensignals (3.4, 0,2), das sich auf das fEKG des einzelnen Fötus bezieht, für mindestens einen Signalkanal diejenige Komponente der digitalisierten Daten innerhalb des Kanals, die dem Fötus zuzuordnen ist, rekonstruiert, die daher einem Einkanal-fEKG entspricht.

4. Gerät nach Anspruch 1, 2 oder 3, bei dem die Signalverarbeitungseinrichtung (34, 36, 46, 47) so ausgebildet ist, dass sie die Daten in digitalisierter Form so verarbeitet, dass mehrere getrennte Quellensignale (62) erzeugt werden, von denen sich mindestens eines (3.4, 0.2) auf das fEKG eines einzelnen Fötus bezieht.

5. Gerät nach einem der Ansprüche 1 bis 4, **dadurch gekennzeichnet, dass** die Elektroden (1-3, R) so auf die Haut aufsetzbar sind, dass die Hauptimpedanz an jeder Elektrode kleiner als 5 kΩ ist.

6. Gerät nach Anspruch 5, **dadurch gekennzeichnet, dass** die Elektroden (1-3, R) so aufsetzbar sind, dass die Hauptimpedanz an jeder Elektrode kleiner als 2 kΩ ist.

7. Gerät nach einem der Ansprüche 1 bis 6, **dadurch gekennzeichnet, dass** die Elektroden mehrere abdominale Elektroden (1-3) zum Aufsetzen auf die Haut (40) im Abdominalbereich sowie eine gemeinsame Referenzelektrode (R) umfassen und die Signalverarbeitungseinrichtung (34, 36, 46, 47) so ausgebildet ist, dass sie Signale aufzeichnet, die Spannungen kennzeichnen, die zwischen jeder abdominalen Elektrode (1-3) und der Referenzelektrode (R) auftreten.

8. Gerät nach Anspruch 7, **dadurch gekennzeichnet, dass** die Elektroden ferner eine rauscharme Elektrode (G) umfassen, die mit Erde verbunden ist, wenn das Gerät verwendet wird.

9. Gerät nach Anspruch 7 oder 8, **dadurch gekennzeichnet, dass** die Signalverarbeitungseinrichtung (34, 36, 46, 47) elektronische Komponenten (34, 46, 47) zur Verarbeitung analoger Spannungssignale, die zwischen zwei Elektroden auftreten, zur Erzeugung digitaler Signale in mehreren Signalkanälen (60) sowie eine Datenverarbeitungseinrichtung (36) aufweist, welche die digitalen Signale verarbeitet.

10. Gerät nach Anspruch 9, **dadurch gekennzeichnet, dass** die elektronischen Komponenten (34, 36, 46, 47) einen rauscharmen Differenzverstärker (37), der die Differenz zwischen dem Spannungssignal jeder Elektrode und einem von der an der Referenzelektrode (R) auftretenden Spannung abgeleiteten Signal verstärkt, ein Glättungs-Tiefpassfilter (38) sowie einen Analog-Digital-Wandler (39) aufweist.

11. Gerät nach Anspruch 10, **dadurch gekennzeichnet, dass** die elektronischen Komponenten in einem von einem Patienten entfernten und zur Elektroenzephalographie (EEG)-Anwendung geeigneten Mehrkanal-Leitungskasten (34) angeordnet sind und die Elektroden (1-3, R) durch die entsprechenden abgeschirmten Leitungen (32a, 32b, 32c, 32e) mit dem Mehrkanal-Leitungskasten (34) verbindbar sind.

12. Gerät nach Anspruch 10 oder 11, **dadurch gekennzeichnet, dass** die elektronischen Komponenten (34, 46, 47) zusätzlich für jede Elektrode (1-3, R) einen entsprechenden Vorverstärker (47) umfassen, der in der Nähe der Elektrode (1-3, R) angeordnet und durch eine entsprechende abgeschirmte Leitung (32a, 32b, 32c, 32e) mit dem Mehrkanal-Leitungskasten (34) verbindbar ist.

13. Gerät nach Anspruch 10, **dadurch gekennzeichnet, dass** die elektrischen Komponenten (34) in einem Leitungskasten (34) untergebracht sind, der in der Nähe eines Patienten positioniert wird, und der Leitungskasten (34) und die Datenverarbeitungseinrichtung (36) so ausgebildet sind, dass sie über eine drahtlose Verbindung (49a, 49b) miteinander kommunizieren.

14. Gerät nach einem der vorhergehenden Ansprüche, **dadurch gekennzeichnet, dass** die Signalverarbeitungseinrichtung (34, 36, 46, 47) Signalkanäle (60) besitzt, die eine Rauschkomponente innerhalb roher, zusammengesetzter Daten von weniger als 10 μV zeigen.

15. Gerät nach Anspruch 14, **dadurch gekennzeichnet, dass** die Signalkanäle (60) eine Signalkomponente von weniger als 5 µV und bevorzugt weniger als 3 µV zeigen.

**16.** Gerät nach einem der vorhergehenden Ansprüche, **dadurch gekennzeichnet, dass** die Signalverarbeitungseinrichtung (34, 36, 46, 47) so ausgebildet ist, dass sie identifiziert, ob sich mehrere Quellensignale (3.4, 0.2) auf einen einzelnen Fötus beziehen oder nicht, und, wenn dies der Fall ist, solche Signale mit einer geeigneten Gewichtung in einer Rekonstruktion des Einkanal-fEKGs kombiniert.

**17.** Gerät nach Anspruch 16 zur Erfassung eines fötalen Elektrokardiogramms im Fall einer Mehrlingsschwangerschaft, **dadurch gekennzeichnet, dass** die Signalverarbeitungseinrichtung (34, 36, 46, 47) so ausgebildet ist, dass sie bei Identifizierung des mindestens einen auf jeden Fötus bezogenen Quellensignals für mindestens eine abdominale Elektrode (1-3) Komponenten der digitalisierten Daten innerhalb des entsprechenden Signalkanals rekonstruiert, die jedem Fötus zuzuordnen sind.

**18.** Gerät nach Anspruch 17, **dadurch gekennzeichnet, dass** die Signalverarbeitungseinrichtung (34, 36, 46, 47) so ausgebildet ist, dass sie für jede abdominale Elektrode (1-3) Komponenten der digitalisierten Daten innerhalb jedes entsprechenden Signalkanals, die jedem Fötus zuzuordnen sind, rekonstruiert und **dadurch** eine Intensitätskarte der Signalstärke von jedem Fötus an der Abdominaloberfläche aufstellt.

**19.** Gerät nach einem der vorhergehenden Ansprüche, **dadurch gekennzeichnet, dass** die Signalverarbeitungseinrichtung (34, 36, 46, 47) so ausgebildet ist, dass sie Quellensignale durch Analyse von unabhängigen Komponenten (Independent Component Analysis, ICA) trennt.

**20.** Gerät nach Anspruch 1, **dadurch gekennzeichnet, dass** die Signalverarbeitungseinrichtung (34, 36, 46, 47) einen Computer (36) aufweist, der so programmiert ist, dass er

a) digitalisierte zusammengesetzte Mehrkanal-Signale filtert, wobei jedes Signal einer Differenz zwischen einer an einer Signalelektrode (1-3) auftretenden Spannung und der an einer Referenzelektrode auftretenden Spannung entspricht, um unerwünschte Frequenzkomponenten zu entfernen,
b) aus der entsprechenden Vielzahl von gefilterten zusammengesetzten Signalen, die jeweils als lineares Gemisch unbekannter Quellensignale ($X = SM$) behandelt werden, eine Vielzahl von getrennten Quellensignalen (62) erzeugt,
c) entweder automatisch oder durch Anforderung einer Benutzer-Eingabe ein Quellensignal oder Quellensignale (3.4, 0.2) erzeugt, die einem EKG eines einzelnen Fötus entsprechen,
d) für jedes identifizierte EKG eines einzelnen Fötus und für mindestens einen Signalkanal diejenige Komponente des gefilterten zusammensetzten Signals innerhalb des Kanals rekonstruiert, die dem Fötus zuzuordnen ist und daher einem Einkanal-fEKG entspricht,
und
e) einem Anwender mindestens ein rekonstruiertes fEKG anzeigt.

## Revendications

**1.** Appareil pour la détection d'un électrocardiogramme foetal (fECG), l'appareil (30, 44, 48) comprenant une pluralité d'électrodes (1-3, R) à appliquer sur un patient et des moyens de traitement du signal (34, 36, 46, 47) pour dériver un fECG à partir de signaux développés par les électrodes (1-3, R), **caractérisé en ce que** :

a) les électrodes sont des électrodes à faible bruit (1-3, R) pour un placement sur la peau abdominale pendant la grossesse et ont des raccordements associés avec un filtrage pour réduire le niveau de bruit et étant en nombre suffisant pour permettre de surveiller au moins six sources de signaux ; et
b) les moyens de traitement du signal sont des moyens de traitement du signal à faible bruit (34, 36, 46, 47), sont raccordables aux électrodes (1-3, R), ont une sensibilité suffisante pour détecter des signaux de magnitude comparables à des signaux d'électroencéphalographie, et sont arrangés pour :

i) détecter des signaux développés par les électrodes ; et
ii) traiter des signaux des électrodes pour obtenir des données et utiliser une technique de Séparation Aveugle des Sources pour traiter les données et distinguer des contributions de sources indépendantes de celles-ci et dériver au moins un signal de source (3.4, 0.2) se rapportant à un seul fECG de foetus.

**2.** Appareil selon la revendication 1, **caractérisé en ce que** les électrodes (1-3, R) sont en nombre suffisant pour permettre de surveiller au moins huit sources de signaux.

**3.** Appareil selon la revendication 1 ou 2, **caractérisé en ce que** les moyens de traitement du signal sont arrangés :

a) pour enregistrer des signaux indiquant des voltages développés entre des paires d'électrodes (1-3, R) dans une pluralité de canaux de signaux (60) ;
b) pour traiter des données numérisées dérivées des voltages d'électrodes afin de générer au moins un signal de source (3.4, 2.0) en rapport avec le fECG d'un seul foetus ; et
c) au moment de l'identification du au moins un signal de source (3.4, 2.0) en rapport avec le fECG d'un seul foetus, pour reconstruire, pour au moins un canal de signaux, cette composante des données numérisées à l'intérieur du canal qui peut être attribuée à ce foetus, et qui en conséquence correspond à un fECG simple canal.

**4.** Appareil selon la revendication 1, 2 ou 3, dans lequel les moyens de traitement du signal (34, 36, 46, 47) sont arrangés pour traiter les données sous forme numérisée pour générer une pluralité de signaux de source séparés (62), dont au moins un (3.4, 2.0) se rapporte au fECG d'un seul foetus.

**5.** Appareil selon l'une quelconque des revendications 1 à 4, **caractérisé en ce que** les électrodes (1-3, R) peuvent être placées sur la peau de sorte que l'impédance de la peau au niveau de chaque électrode est inférieure à 5 kΩ.

**6.** Appareil selon la revendication 5, **caractérisé en ce que** les électrodes (1-3, R) peuvent être placées sur la peau de sorte que l'impédance de la peau au niveau de chaque électrode est inférieure à 2 kΩ.

**7.** Appareil selon l'une quelconque des revendications 1 à 6, **caractérisé en ce que** les électrodes comprennent une pluralité d'électrodes abdominales (1-3) pour un placement sur la peau (40) dans la région abdominale et une électrode de référence commune (R), et les moyens de traitement du signal (34, 36, 46, 47) sont arrangés pour enregistrer des signaux indiquant des voltages développés entre chaque électrode abdominale (1-3) et l'électrode de référence (R).

**8.** Appareil selon la revendication 7, **caractérisé en ce que** les électrodes comprennent aussi une électrode à faible bruit (G) qui est raccordée à la terre quand l'appareil est utilisé.

**9.** Appareil selon la revendication 7 ou 8, **caractérisé en ce que** les moyens de traitement du signal (34, 36, 46, 47) comprennent des composants électroniques (34, 46, 47) pour traiter des signaux de voltage analogiques développés entre des paires d'électrodes pour fournir des signaux numériques dans une pluralité de canaux de signaux (60) et des moyens de traitement des données (36) pour traiter les signaux numériques.

**10.** Appareil selon la revendication 9, **caractérisé en ce que** les composants électroniques (34, 46, 47) intègrent un amplificateur différentiel à faible bruit (37) pour amplifier la différence entre le signal de voltage de chaque électrode et un signal dérivé du voltage développé au niveau de l'électrode de référence (R), un filtre anti-repliement passe-bas (38) et un convertisseur analogique-numérique (39).

**11.** Appareil selon la revendication 10, **caractérisé en ce que** les composants électroniques sont situés dans une boîte de raccordements multicanaux (34) éloignée d'un patient et convenable pour un usage électroencéphalographique (EEG), et les électrodes (1-3, R) peuvent être raccordées à la boîte de raccordements multicanaux (34) par des raccordements filtrées respectives (32a, 32b, 32c, 32e).

**12.** Appareil selon la revendication 10 ou 11, **caractérisé en ce que** les composants électroniques (34, 46, 47) comprennent en outre, pour chaque électrode (1-3, R), un pré-amplificateur respectif (47) situé adjacent à cette électrode (1-3, R) et pouvant être raccordé à la boîte de raccordements multicanaux (34) par un raccordement filtré respectif (32a, 32b, 32c, 32e).

**13.** Appareil selon la revendication 10, **caractérisé en ce que** les composants électroniques (34) sont situés dans une boîte de raccordements (34) pour un positionnement adjacent à un patient, et la boîte de raccordements (34) et les moyens de traitement du signal (36) sont arrangés pour communiquer par une liaison sans fil (49a, 49b).

**14.** Appareil selon l'une quelconque des revendications précédentes, **caractérisé en ce que** les moyens de traitement du signal (34, 36, 46, 47) ont des canaux de signaux (60) qui montrent une composante de bruit à l'intérieur de données composites brutes inférieure à 10 µV.

**15.** Appareil selon la revendication 14, **caractérisé en ce que** les canaux de signaux (60) montrent une composante de bruit inférieure à 5 µV et de préférence inférieure à 3µV.

**16.** Appareil selon l'une quelconque des revendications précédentes, **caractérisé en ce que** les moyens de traitement du signal (34, 36, 46, 47) sont arrangés pour identifier si une pluralité de signaux de source (3.4, 2.0) se rapportent ou non à un seul foetus, et le cas échéant pour combiner de tels signaux avec une pondération appropriée dans une reconstruction du fECG simple canal.

**17.** Appareil selon la revendication 16 pour la détection d'électrocardiogrammes foetaux dans une grossesse multiple, **caractérisé en ce que** les moyens de traitement du signal (34, 36, 46, 47) sont arrangés, au moment de l'identification du au moins un signal de source en rapport avec chaque foetus, pour reconstruire, pour au moins une électrode abdominale (1-3), des composantes des données numérisées à l'intérieur du canal de signaux correspondant pouvant être attribuées à chaque foetus.

**18.** Appareil selon la revendication 17, **caractérisé en ce que** les moyens de traitement du signal (34, 36, 46, 47) sont arrangés pour reconstruire, pour chaque électrode abdominale (1-3), des composantes des données numérisées à l'intérieur de chaque canal de signaux correspondant qui peuvent être attribuées à chaque foetus et de cette manière pour construire une carte d'intensité de la surface abdominale de la force du signal de chaque foetus.

**19.** Appareil selon l'une quelconque des revendications précédentes, **caractérisé en ce que** les moyens de traitement du signal (34, 36, 46, 47) sont arrangés pour séparer les signaux de source par une Analyse en Composantes Indépendantes (ACI).

**20.** Appareil selon la revendication 1, **caractérisé en ce que** les moyens de traitement du signal (34, 36, 46, 47) incorporent un ordinateur (36) programmé pour :

a) filtrer des signaux composites numérisés multicanaux, chaque signal correspondant à une différence entre un voltage développé au niveau d'une électrode de signal (1-3) et celui développé au niveau d'une électrode de référence (R), afin de supprimer des composantes de fréquence non désirées ;
b) générer une pluralité de signaux de source séparés (62) à partir de la pluralité de signaux composites filtrés correspondants, chacun étant traité comme un mélange linéaire de signaux de sources inconnus (X = SM) ;
c) identifier, soit automatiquement ou en demandant une saisie d'un utilisateur, un signal de source ou des signaux de source (3.4, 2.0) correspondant à un ECG d'un seul foetus;
d) reconstruire, pour chaque ECG d'un seul foetus identifié, et pour au moins un canal de signaux, cette composante du signal composite filtré à l'intérieur du canal qui peut être attribuée à ce foetus et en conséquence correspond à un fECG simple canal ; et
e) montrer au moins un fECG reconstruit à un utilisateur.

**Figure 1**

**Figure 2**

Figure 3

Figure 4

**Figure 5a**

EP 1 432 349 B1

**Figure 5b**

EP 1 432 349 B1

**Figure 6**

Figure 7

27

Figure 8

**Figure 9**